# EUROPEAN PATENT SPECIFICATION

(11) **EP 3 968 755 B1**
(45) Date of publication and mention of the grant of the patent: **23.08.2023**
(21) Application number: 20735451.5
(22) Date of filing: 14.05.2020
(51) Int. Cl.: A01G 7/04, A01G 9/26

(54) **PLANT ILLUMINATION METHOD AND SYSTEM**
VERFAHREN UND SYSTEM ZUR PFLANZENBELEUCHTUNG
PROCÉDÉ ET SYSTÈME D'ÉCLAIRAGE DE VÉGÉTAUX

(30) Priority: 15.05.2019 PL 42993919; 26.07.2019 PL 43073319; 02.01.2020 PL 43249420
(43) Date of publication of application: 23.03.2022
(73) Proprietor: Trawinski, Andrzej, 51-180 Psary (PL)
(72) Inventor: Trawinski, Andrzej, 51-180 Psary (PL)
(74) Representative: JWP Patent & Trademark Attorneys
(86) International application number: PCT/IB2020/054568
(87) International publication number: WO 2020/230073

(56) References cited:
- WO-A1-2008/118080
- WO-A1-2011/115123
- WO-A1-2019/031559
- WO-A2-00/54567
- US-A1- 2013 263 503

## Description

The present invention relates to a method and system for illuminating plants, in particular plants intended for greenhouse cultivation (including annual plants), the method and the system being based on a modulated light source. The objects of the invention are applied in agriculture, and particularly in greenhouse cultivation and undercover cultivation.

Luminous intensity and light spectrum are factors which determine the cellular processes in plants, as well as plant growth and development. Plants have photoreceptors which precisely receive light of a particular wavelength - until present 4 such receptors have been described. Red light and blue light have the greatest influence on plant growth, as they are the main source of energy required for the assimilation of CO₂ in the process of photosynthesis (Lin et al. 2013. Sci. Hort. 150:86-91). Furthermore, red light is necessary for plant development, including the development of the photosynthetic apparatus (Paradiso et al. 2011. Acta Hortic., 893: 849-55). Blue light regulates among other things chlorophyll biosynthesis and chloroplast development, as well as stomata movement and photomorphogenesis (Demarsy and Frankhauser 2009. Curr. Opin. Plant Biol. 12:69-74). UV light may improve resistance to disease and change the color of leaves. Light modifies the chemical composition of the plant - of both its vegetative part and the seeds or fruits. Light of a particular spectrum, adjusted to the cultivated species or to the development phase of the plant, is increasingly often used in special cultivation systems, with fully controlled thermal and light conditions. In this case, light is produced by energy-saving LED (Light-Emitting Diodes) modules. In order to effectively use LED modules in horticulture, it is an increasingly frequent practice to match the spectral characteristics of the light sources to the requirements of photosynthesis and photomorphogenesis of a particular species. Much less attention has been until present given to the luminous intensity of the lamps, and as a result this intensity remains constant throughout the day for greenhouse-cultivated crops illuminated in the fall-winter period. In nature, however, luminous intensity varies throughout the day and in addition the entire plants or their individual parts are subjected to temporary shading.

Prior art knows plant illumination methods and systems which operate by providing illumination in a constant or in a pulse manner and in which the pulse illumination is especially realized by a cyclical, discrete switching of a lamp system from a switched-on state to a switched-off state and from a switched-off state to a switched-on state. The illumination pulse lasts for approximately 1 second, and the time during which the illumination is switched off typically varies from approximately 1 second to several seconds. Typically, illumination systems make use of fixtures based on LED source of light as well as HPS-type (High Pressure Sodium) lamps. A plant illumination method and system is known from document US 2013/263503 A1.

Document WO 0054567 A2 discloses an apparatus and a method for promoting the germination of plant seeds and the growth of plants. The wavelength of the radiation emitted towards the plants remains within the visible range of wavelengths and is approximately 400 - 700 nm. The light may change in pulses at a frequency of about 10 to 150 pulses per minute, with each light pulse having a duration of 0.1 - 0.9 sec. The intervals between the pulses, i.e. the time periods when the lamps are switched off, have a duration of 0.1 to 6 seconds. The apparatus comprises a means for directing a series of light pulses at the seeds and plants, as well as a means for controlling the duration of the pulses and the duration of the intervals between the pulses.

European patent document EP3127421 discloses a device and a method for promoting the growth of a plant with the use of pulse light. The solution operates by alternately illuminating the plants with a red light pulse having a wavelength of 600 - 680 nm and a far red light or an infrared light having a wavelength of 700 - 900 nm. The plants may be illuminated continuously, but with the use of different wavelengths. The device produces the red light and the infrared light using two types of LED light sources, which are alternately turned on.

International patent document WO 2019060788 A1 discloses a method for energizing a plurality of light channels of a horticulture lighting device. Plants are illuminated by switching the lamps on and off in cycles in order to trigger biochemical reactions in plants during the photosynthesis process. The system illuminates plants in pulses at a frequency of 120 Hz to 720 Hz, which may be unperceivable to a human eye. The pulse delay, or an off period in which the lamps are switched off, is of 500 µs to 3 ms. The frequency of the light pulses is controlled by a PWM controller, which regulates the duty cycle. The disclosed solution makes use of at least two LED devices, each of them emitting light of a different wavelength.

The technical problem of the present invention is to offer such a method and a system for plant illumination which will provide the best effects for the development of seeds and seedlings, as well as for the development of plants during their growth and fructification. It is also desired that the plant illumination method and system provides energy savings and contributes to increased lamp life in the system. It is further desired that the plant illumination method and system can be implemented in already existing illumination systems.

The first object of the present invention is a plant illumination method according to claim 1, in which light pulses are delivered to the plants in cycles from a light source at any stage of plant development, characterized in that one illumination cycle comprises a bright pulse having a duration time t₁ and a dark pulse having a duration time t₂, whereby the illumination frequency is in the range of 2 cycles/minute to 6 cycles/minute, and the ratio between the bright pulse duration time t₁ and the duration time of the cycle t₁+t₂ is in the range of 0.25 to 0.5.

In the preferred embodiment of the present invention, the bright pulse has an intensity corresponding to 90%-100% of the maximum power of the light source, and the dark pulse has an intensity corresponding to 7% - 30% of the maximum power of the light source.

In another preferred embodiment of the invention, the light source maximum power emits a photosynthetic photon flux of 680 µmol/s.

In another preferred embodiment, the daily photoperiod is in the range of 12 hours to 24 hours.

Preferably, the photosynthetic photon flux density of the light source is in the range of 216 to 370 µmol/m²/s.

Also preferably, the bright pulse duration time t₁ is 4 seconds and the dark pulse duration time t₂ is 8 seconds, or the bright pulse duration time t₁ is 8 seconds and the dark pulse duration t₂ is 8 seconds, or the bright pulse duration time t₁ is 4 seconds and the dark pulse duration time t₂ is 12 seconds, or the bright pulse duration time t₁ is 5 seconds and the dark pulse duration time t₂ is 5 seconds, or the bright pulse duration time t₁ is 10 seconds and the dark pulse duration time t₂ is 20 seconds, or the bright pulse duration time t₁ is 5 seconds and the dark pulse duration time t₂ is 8 seconds.

In a preferred embodiment of the invention, light emitted from the light source has a wavelength in the range of 380 to 780 nm.

In another preferred embodiment, the illuminated plants are plants intended for greenhouse cultivation. Preferably, the plants comprise all varieties of chrysanthemums, tomatoes, basil and sweet pepper.

Preferably, plant illumination pulses have a substantially rectangular waveform.

The second object of the present invention is a plant illumination system according to claim 8, comprising at least one light source connected to a power supply unit which includes a control means for providing cyclical operation of the light source, characterized in that one illumination cycle comprises a bright pulse having a duration time t₁ and a dark pulse having a duration time t₂, whereby the illumination frequency is in the range of 2 cycles/minute to 6 cycles/minute, and the ratio between the bright pulse duration time t₁ and the duration time of the cycle t₁+t₂ is in the range of 0.25 to 0.5.

In a preferred embodiment of the invention, the light source is a LED lamp.

In another preferred embodiment of the present invention, the bright pulse has an intensity corresponding to 90% - 100% of the maximum power of the light source, and the dark pulse has an intensity corresponding to 7% - 30% of the maximum power of the light source.

In another preferred embodiment of the invention, the light source maximum power emits a photosynthetic photon flux of 680 µmol/s.

Preferably, the daily photoperiod is in the range of 12 hours to 24 hours.

Also preferably, the photosynthetic photon flux density of the light source is in the range of 216 to 370 µmol/m²/s.

Also preferably, the bright pulse duration time t₁ is 4 seconds and the dark pulse duration time t₂ is 8 seconds, or the bright pulse duration time t₁ is 8 seconds and the dark pulse duration time t₂ is 8 seconds, or the bright pulse duration time t₁ is 4 seconds and the dark pulse duration time t₂ is 12 seconds, or the bright pulse duration time t₁ is 5 seconds and the dark pulse duration time t₂ is 5 seconds, or the bright pulse duration time t₁ is 10 seconds and the dark pulse duration time t₂ is 20 seconds, or the bright pulse duration time t₁ is 5 seconds and the dark pulse duration time t₂ is 8 seconds.

In a preferred embodiment of the invention, light emitted from the light source has a wavelength in the range of 380 to 780 nm.

In another preferred embodiment, the illuminated plants are plants intended for greenhouse cultivation. Preferably, the plants comprise all varieties of chrysanthemums, tomatoes, basil and sweet pepper.

Preferably, plant illumination pulses have a substantially rectangular waveform.

The method and system for plant illumination by cyclically, alternately delivering bright and dark light pulses to the plants provides better effects for the development of seeds and seedlings, as well as for the development of plants during their growth and fructification. Lamps operating in a pulse mode consume less energy and are more durable, offering longer life without the need for servicing or replacing them.

The authors of the present invention concluded that plants, owing to their adaptability, receive an amount of energy sufficient to initiate photosynthesis, as in the case of continuous delivery of light energy. The method for plant illumination according to the present invention allows significant savings of electric energy, at 60% - 80% in comparison to continuous HPS illumination and at 40% - 50% in comparison to continuous LED illumination.

Advantageously, the invention provides an effect of incomplete switching off, a fluent transition to lower and higher powers of the light energy emitted by the light source, and also allows white colorto be perceived by the human eye, so that workers in greenhouses can work in free and ergonomic conditions at modulated semiconductor light source.

Exemplary embodiments of the invention are presented in the drawing, in which Fig. 1 is a block diagram of a plant illumination system according to the invention, Fig. 2 is a comparative photograph of "Lena" pepper in its 55^{th} day of vegetation under the plant illumination systems (PULS1-5) and under a reference system (HPS), Fig. 3 is a comparative photograph of "Lettuce-leaved" basil in its 55^{th} day of vegetation under the plant illumination systems (PULS1-5) and under a reference system (HPS), Fig. 4 is a graph showing the germination dynamics of Lena pepper in the illumination system according to the invention with the use of illumination parameters PULS1, PULS6 and PULS7, Fig. 5 is a comparative photograph of Lena pepper in its 55^{th} day of vegetation under the plant illumination systems (PULS1, PULS6 and PULS7), Fig. 6 is a graph showing the germination dynamics of Lettuce-leaved basil in the illumination system according to the invention with the use of illumination parameters PULS1, PULS6 and PULS7, Fig. 7 is a comparative photograph of Lettuce-leaved basil in its 55^{th} day of vegetation under the plant illumination systems (PULS1, PULS6 and PULS7), Fig. 8 is a spectral characteristic of the light source in the plant illumination system, Fig. 9 shows the substantially rectangular waveform of the illumination control signal in the illumination system according to the invention for a preferred embodiment (PULS2).

### Example 1

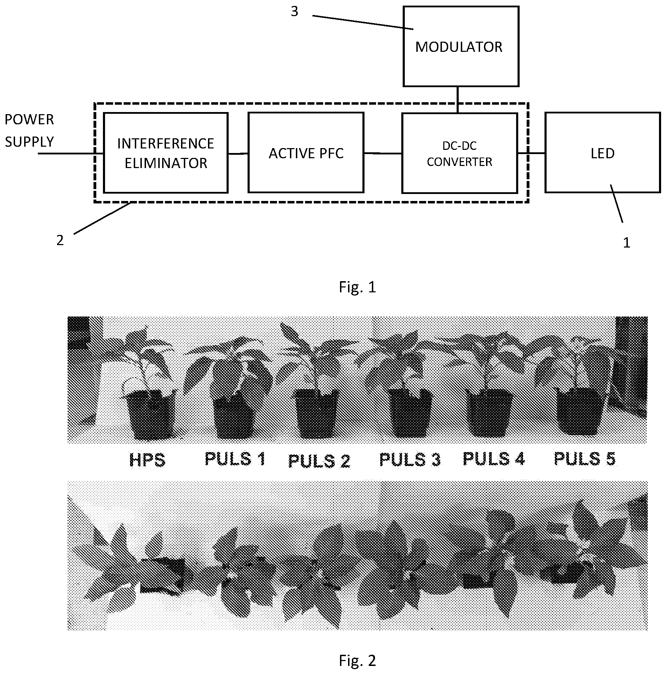

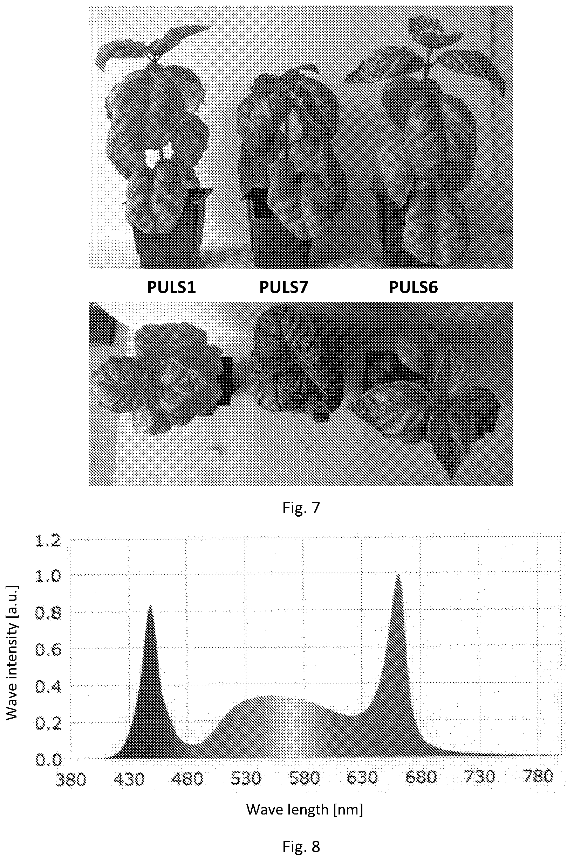

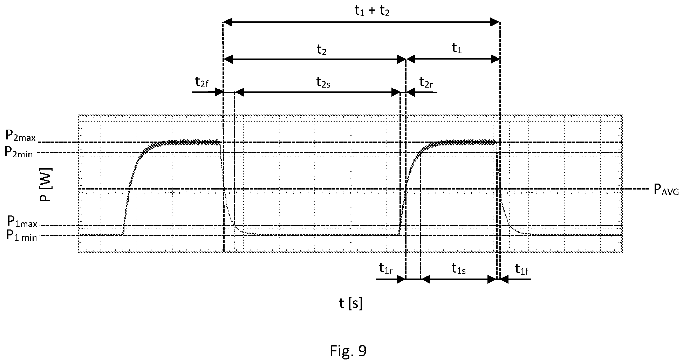

The plant illumination system according to the first embodiment of the invention has been shown schematically in Fig. 1. In general, the system comprises a power supply system 2, a light source 1 and control means 3. The light source 1 comprises a fixture made of aluminum profiles, which house the main LED module (together with the printed circuit board and with the light-emitting diodes). The power supply system 2 consists of two converters, the first converter being a converter with an active PFC circuit, and the second converter being a DC-DC regulated converter controlled by control means 3 comprising a luminous intensity regulating module. The light source 1 emits electromagnetic waves in the visible light range, i.e. of 380 nm to 780 nm, having a spectral characteristic shown in Fig. 8. Such a spectral characteristic was obtained by the appropriate selection of LEDs. A white diode 6500 K was used in combination with a red diode (Hyper RED 660 nm) and with a blue diode (Deep BLUE 450 nm) in various proportions. The system allows providing any shape to the light pulses delivered to plants, in the operating cycles described below. Fig. 9 shows the substantially rectangular waveform of the illumination control in the illumination system according to the invention for a preferred embodiment (PULS2). The illumination control waveform is a waveform characteristic of RC circuits, in which an adequately selected capacity influences the rise and fall characteristics of the control signal. By selecting an appropriately small RC time constant, it is possible to adjust the control waveform even closer to the rectangular waveform.

Importantly, the waveform shown in Fig. 9, the bright pulse duration time t₁ and the dark pulse duration time t₂ are calculated with respect to the value P_{AVG}, which is the power median (P₂ₘₐₓ - P₁ₘᵢₙ)/2. Therefore, with the characteristic waveform of RC circuits taken into consideration, the bright pulse duration time t₁ includes the following components: the bright pulse rise time t₁ᵣ, in which the light source power rises from the value P_{AVG} to the value P₂ₘᵢₙ, the bright pulse saturation time t₁ₛ, in which the light source power is kept within the range P₂ₘᵢₙ - P₂ₘₐₓ (in this embodiment, P₂ₘᵢₙ = P₂ₘₐₓ - 10% (P₂ₘₐₓ - P₁ₘᵢₙ)), the bright pulse fall time tif, in which the light source power falls from the value P₂ₘᵢₙ to the value P_{AVG}. Analogically, the dark pulse duration time t₂ includes the following components: the dark pulse fall time t_{2f}, in which the light source power falls from the value P_{AVG} to the value P₁ₘₐₓ, the dark pulse saturation time t₂ₛ, in which the light source power is kept within the range P₁ₘᵢₙ - P₁ₘₐₓ (in this embodiment, P₁ₘₐₓ = P₁ₘᵢₙ + 10% (P₂ₘₐₓ - P₁ₘᵢₙ)), the dark pulse rise time t₂ᵣ, in which the light source power rises from the value P₁ₘₐₓ to the value P_{AVG}. The more the waveform approaches a rectangular shape, the smaller the contribution of the rise times and the fall times to the illumination characteristics. For clarity purposes, the notion of bright pulse duration time t₁ used in this description covers the above rise, saturation and fall times for the bright pulse, and the notion of dark pulse duration time t₂ covers the above rise, saturation and fall times for the dark pulse.

Tests were performed to evaluate the growth and the efficiency of the bright phase of photosynthesis (PSII) for pepper and basil cultivated under the plant illumination system according to the invention. The tested plants were one variety of Lettuce-leaved basil and one variety of Lena pepper, both dedicated for undercover cultivation.

Each group of plants was illuminated with the use of the plant illumination system according to the invention, and the parameters of the used illumination cycles are provided in Table 1. A classic system comprising a light source in the form of HPS lamps was used as a reference.

The tests were performed in closed, isolated vegetation chambers in controlled thermal conditions: temperature 25 °C ± 1°C, photoperiod: 12h day/12h night. Table 1 also shows irradiation intensity on the surface of the crop field (defined as photosynthetic photon flux density- PPFD) for each plant illumination system. The seeds were sowed into a moist soil (the mixture: Ekoziem Universal Substrate for flowers and vegetables pH 6-7 (5L) + Substral Osmocote pH 5.4-6 (20L) + sand (1.5 kg)).

**Table 1- parameters of the applied illumination**

| | **P1 [W]** | **P₂ [W]** | **t₁ [s]** | **t₂ [s]** | **f [cycles/min]** | **t_{1/}(t₁+t₂)** | **PPFD [µmol/m²/s]** |
|---|---|---|---|---|---|---|---|
| **HPS** | 400 | | continuous operation | | | | 280 |
| **PULS1** | 200 | 20 | 4 | 8 | 5 | 0.33 | 370 |
| **PULS2** | 140 | 10 | 8 | 8 | 3.75 | 0.5 | 216 |
| **PULS3** | 200 | 20 | 4 | 12 | 3.75 | 0.25 | 270 |
| **PULS4** | 140 | 10 | 5 | 5 | 6 | 0.5 | 247 |
| **PULS5** | 200 | 20 | 10 | 20 | 2 | 0.33 | 327 |

where:
- P₁: - Bright pulse power,
- P₂: - Dark pulse power,
- t₁: - Bright pulse duration time,
- t₂: - Dark pulse duration time,
- T: - Number of illumination cycles per minute,
- PPFD: - photosynthetic photon flux density of the light source.

Importantly, the PPFD values provided in Table 1 are average values for the entire illumination cycle, which covers the bright pulse having duration time t₁ and the dark pulse having duration time t₂. In addition, the power of a light source should not be understood as a limiting parameter either, because as such it depends on the technology used in a particular light source. In this particular field of the invention, the parameter important for achieving desired plant growth is understood as the parameter describing the density of photon flux reaching the surface of a plant, provided in micromoles (µmol) per square meter per second (PPFD). Therefore, the light sources of the present invention must emit a light which is spectrally adjusted to the growth of a particular plant and provide an adequate photosynthetic photon flux to the surface of an illuminated plant. Regardless of the used light source power and of the distance from the light source to the plant surface receiving the photosynthetically effective electromagnetic radiation, a photon flux density of above 200 µmol/m²/s should be provided on the plant surface, and therefore alternative embodiments may use light sources of a greater or smaller power, hung at various heights over the surface of the illuminated plant. In this embodiment, a light source having a power of 200 W provided a photon flux of 680 µmol/s, a light source having a power of 20 W provided a photon flux of 59 µmol/s, a light source having a power of 140 W provided a photon flux of 476 µmol/s, and a light source having a power of 10 W provided a photon flux of 29 µmol/s.

In the case of both basil and pepper, further biometric measurements, including plant height measurement, were performed in subsequent development phases (for the seedlings of the plant plugs and for the plants in their 55^{th} day of vegetation). The plant height was measured from the base of the stem to the tip of the plant. Simultaneous morphological observations were performed, and the description included the color of leaves, the condition of the plants, and - in the case of the pepper - also the number of leaves and flowers in its 55^{th} day of vegetation. Photographic documentation was performed.

At the end of the experiment (55^{th} day of vegetation), the above-ground parts of basil and pepper were collected and weighed in order to measure the accumulation of fresh mass.

Greenness intensity of plant leaves were measured for the basil and pepper plug plants with a SPAD Minolta camera. Leave greenness intensity is an indirect measure of the content of photosynthetic dyes from the chlorophyll group. A conventionally accepted unit is the absolute SPAD value.

A chlorophyll fluorescence measurement was performed with the use of Plant Efficiency Analyzer in order to estimate the efficiency of photosystem II (PSII) (PEA, Hansatech Ltd., King's Lynn, UK). Details of the procedure are provided by Skoczowski et.al. (2011). PEA, which uses the analysis of the fast kinetics of chlorophyll *a* fluorescence, is a convenient tool for describing a great number of photosynthetic variables and the efficiency characteristics of reactions which take place within the bright phase of photosynthesis as part of photosystem II. The obtained fluorescence curve serves to calculate parameters which reflect how effectively the photosystem functions.

The calculations also included the so-called performance index (a parameter which describes the general efficiency of the photosystem - PI abs), which is an overall (single-number) representation of the general efficiency of photosystem II in relationship to absorption (the calculation formula available in Strasser et al. 2000). The measurement was performed in 8 - 12 repetitions (on 8-12 plants) for a single object (lamp). The measurement was performed on leaf No. 1 for pepper (plug plant phase L1), and on leaf No. 1 for basil (plug plant phase L1). Additionally, basic fluorescence parameters of chlorophyll *a* were estimated in the form of structure indicators and functions, i.e. Fv/Fo - maximum water splitting efficiency on the donor side of PSII. The parameter characterizes the efficiency of water splitting in PSII and thus, the efficiency of oxygen release. These estimations were performed for Lena pepper and for Lettuce-leaved basil, for the first leaf (L1) in the plug plant phase and for the fifth leaf (L5) in the 55^{th} day of vegetation.

The above-described measurement data are provided in Table 2 for Lena pepper and in Table 3 for Lettuce-leaved basil.

**Table 2 - measurement data for Lena pepper**

| | **HPS** | **PULS1** | **PULS2** | **PULS3** | **PULS4** | **PULS5** |
|---|---|---|---|---|---|---|
| **hᵣ [cm]** | 1.55 | 2.12 | 2.08 | 2.31 | 2.45 | 2.82 |
| **IZ (L1r) [SPAD]** | 25.96 ± 1.67 | 30.32 ± 3.12 | 27.42 ± 3.50 | 30.10 ± 3.92 | 28.59 ± 3.92 | 27.34 ± 4.15 |
| **hw [cm]** | 4.59 | 5.63 | 4.59 | 5.13 | 5.61 | 6.34 |
| **mw [g]** | 3.04 | 3.84 | 3.42 | 3.36 | 3.75 | 3.97 |
| **PI abs (L1ᵣ)** | 2.4 | 3.58 | 4.01 | 4.42 | 4.05 | 3.86 |
| **F_{V}/F₀ (L1ᵣ)** | 3.2 | 3.4 | 3.63 | 3.72 | 3.69 | 3.61 |
| **Fᵥ/F₀ (L5_{W})** | 3.01 | 3.11 | 3.18 | 3.26 | 3.46 | 3.47 |

where:
hᵣ - plant height for the Lena pepper plug plant,
IZ (L1ᵣ) - leave greenness intensity for the Lena pepper plug plant (first leaf - L1),
h_{w} - plant height for Lena pepper in its 55^{th} day of vegetation,
m_{w} - fresh mass (biomass accumulation) for Lena pepper in its 55^{th} day of vegetation,
PI abs (L1ᵣ) - performance index value of photosystem II for the first leaf (L1) of Lena pepper in the plug plant phase,
Fv/Fo (L1ᵣ) - maximum water splitting efficiency on the donor side of PSII for the first leaf (L1) of Lena pepper in the plug plant phase,
F_{V}/F₀ (L5_{w}) - maximum water splitting efficiency on the donor side of PSII for the fifth leaf (L5) of Lena pepper in its 55^{th} day of vegetation.

Referring to Table 2, the highest plug plants were obtained under a plant illumination system with PULSS.were The plants cultivated under the HPS lamp were significantly shorter than the plants cultivated in the plant illumination systems of the present invention (PULS1-5). In addition, during the plug plant phase the highest SPAD values (the most intensive greenness) were observed for the plants cultivated under the plant illumination system of the invention, in illumination conditions PULS1 and PULS3, while the lowest value was observed for plants illuminated with the HPS-lamp system. Moreover, in the 55^{th} day of vegetation, the highest pepper plants were found in the plant illumination system with the use of PULS5 illumination, while the shortest plants were obtained in the plant illumination system with the use of PULS5 illumination and in the reference HPS system. Plants from the plant illumination system with the parameters defined as PULS1, PULS3 and PULS4 accounted for intermediate values, but higher than observed in plants illuminated with the reference HPS system. Additionally, in the 55^{th} day of vegetation, the plants cultivated in the plant illumination system with parameters PULS1, PULS4 and PULS5 showed the greatest accumulation of fresh mass, while the pepper plants under the reference HPS-based illumination system showed the smallest accumulation of fresh mass.

Peppers (plug plant phase, first leaf L1) cultivated under any combination of illumination parameters PULS1-5 in the plant illumination system according to the invention had leaves showing higher (49 - 84%) performance index values of the photosystem II in relationship to absorption (PI abs) as compared to the leaves of plants cultivated in the reference HPS system. Based on additional fluorescence parameters of chlorophyll a, it may be also concluded that the type of the applied plant illumination system and illumination parameters have a significant influence on the photosynthesis process (the bright phase) in the plug plant phase. This fact is exemplified by the parameter F_{v/}F₀, which describes the amount of the oxygen released during the photosynthesis process. The highest value of this parameter, and thus the greatest oxygen release, was observed for plants in the plug plant phase cultivated under the plant illumination system according to the invention, with the use of illumination parameters PULS3 and PULS4, while the smallest oxygen release was observed for plants illuminated in the reference HPS-lamp system. After 55 days of vegetation, similar relationships may be observed: oxygen releases for plants cultivated under the plant illumination system according to the invention were higher than the release observed in the reference HPS-lamp system.

Fig. 2 shows a comparative photograph of Lena pepper in its 55^{th} day of vegetation under different plant illumination systems, i.e. the plant illumination systems according to the invention (PULS1-5) and the reference (HPS) system. The plants in their 55^{th} day of vegetation were observed to have various numbers of leaves; HPS: 10 large leaves, PULS1: 12 large leaves and primordia of lateral shoots, PULS2: 9 large and 4 small leaves, PULS3 and PULS5: 11 large leaves, PULS4: 14 large leaves. A difference in the number of flowers was also observed. Number of flowers provided as an average based on observations of three plants - HPS: none, PULS1: 1 flower and 6 buds, PULS2: 1 flower and 7 buds, PULS3: 1 flower and 4 buds, PULS4: 2 flowers and 4 buds, PULS5: 3 flowers and 5 buds.

The fact that flowers developed in the plant illumination systems according to the invention (unlike in the reference HPS-lamp system) indicates an increased growth rate of pepper in the illumination conditions of the present invention. The number of flowers and buds may indicate the number of fruits that the plant will be able to develop. Fruits will be available at the soonest (and in the greatest number) in the pepper cultivated under the plant illumination system with the use of illumination parameters PULS5. In the reference HPS system, the pepper needs longer time to develop flower buds and it may take longer time to develop fruits.

In sum, the plant illumination system according to the invention, with the use of illumination parameters PULS1-5, positively influenced the photosynthetic efficiency and the growth of pepper plug plants in comparison with the reference HPS-lamp system. At the plug plant stage, all of the illumination parameters PULS1-5 provided better effects than the effects obtained with the reference HPS system. At further pepper development stages, the application of the plant illumination system according to the invention with the use of illumination parameters PULS1-5 accelerated plant growth, this fact being demonstrated by an earlier (in comparison to the reference HPS system) development of flowers on the plants. The plants in the plant illumination systems according to the invention also developed more leaves and generally accumulated more fresh mass.

**Table 3 - measurement data for Lettuce-leaved basil**

| | **HPS** | **PULS1** | **PULS2** | **PULS3** | **PULS4** | **PULS5** |
|---|---|---|---|---|---|---|
| **hᵣ [cm]** | 4.64 | 10.57 | 5.32 | 7.14 | 5.96 | 8.19 |
| **IZ (L1ᵣ) [SPAD]** | 17.84 ± 4.02 | 18.15 ± 1.63 | 20.03 ± 2.27 | 18.69 ± 1.64 | 20.76 ± 3.58 | 19.70 ± 1.66 |
| **h_{w} [cm]** | 12.31 | 17.44 | 11.65 | 14.08 | 15.31 | 18.01 |
| **m_{w} [g]** | 8.14 | 12.24 | 8.48 | 9.05 | 10.36 | 9.2 |
| **PI abs (L1ᵣ)** | 1.3 | 3 | 3.1 | 2.8 | 3.8 | 3.3 |
| **F_{V}/F₀ (L1ᵣ)** | 3.93 | 4.85 | 4.48 | 4.62 | 4.64 | 4.53 |
| **F_{V}/F₀ (L4_{W})** | 2.44 | 4.35 | 4.07 | 3.42 | 3.52 | 4.38 |

where:
hᵣ - plant height for the Lettuce-leaved basil plug plant,
IZ (L1ᵣ) - leave greenness intensity for the Lettuce-leaved basil plug plant (first leaf - L1),
h_{w} - plant height for Lettuce-leaved basil in its 55^{th} day of vegetation,
m_{w} - fresh mass (biomass accumulation) for Lettuce-leaved basil in its 55^{th} day of vegetation,
PI abs (L1ᵣ) - performance index value of photosystem II for the first leaf (L1) of Lettuce-leaved basil in the plug plant phase,
Fv/Fo (L1ᵣ) - maximum water splitting efficiency on the donor side of PSII for the first leaf (L1) of Lettuce-leaved basil in the plug plant phase,
Fv/Fo (L4_{w}) - maximum water splitting efficiency on the donor side of PSII for the leaf of the 4^{th} level (L4) of Lettuce-leaved basil in its 55^{th} day of vegetation.

In reference to Table 3, the highest plants of Lettuce-leaved basil were obtained in the plant illumination system according to the invention with the use of illumination parameters PULS1, and the plants of the plug plants obtained in the reference HPS-lamp system were significantly shorter (by more than 50%). Intermediate values (although significantly higher than in the case of the plants cultivated in the reference HPS system) were obtained by the plants cultivated in the plant illumination system with the use of illumination parameters PULS3 and PULS5. Referring to the leave greenness intensity in Lettuce-leaved basil (the value provided in conventional SPAD units), the highest SPAD values (the most intensive greenness) in the plug plant phase were observed for the L1 of the plants grown in the plant illumination system according to the invention with the use of illumination parameters PULS4. In the case of the plug plant grown in the reference HPS-lamp system, and especially in the case of L1, the SPAD values were the lowest. On the other hand, with reference to the height of Lettuce-leaved basil in its 55^{th} day of vegetation, the highest plants of Lettuce-leaved basil were obtained in the plant illumination systems with the use of illumination parameters PULS1 and PULS5, while the shortest plants, albeit comparable to those from the reference HPS-lamp system, were obtained for illumination parameters PULS2. The plants obtained with the use of the remaining illumination parameters reached intermediate values, albeit statistically significantly higher than in the case of the plants cultivated in the reference HPS system.

Measurements of fresh mass in the above-ground parts (biomass accumulation) of Lettuce-leaved basil in its 55^{th} day of vegetation indicate that Lettuce-leaved basil grown in the plant illumination system with the use of illumination parameters PULS1 accumulated the most biomass, reaching high values of fresh mass in the above-ground parts (the values were significantly higher than in the reference HPS-lamp system). A relatively high (also higher than in the HPS reference system) accumulation of fresh mass was observed also in the case of the plants in the plant illumination system according to the invention with the use of illumination parameters PULS4.

In the case of the Lettuce-leaved basil, the measurements were performed on the first leaf in the plug plant phase. In the case of the first leaves of the basil plants cultivated under the plant illumination systems according to the invention (for illumination parameters PULS1-5), observations demonstrated higher performance index values of photosystem II in relation to absorption (PI abs) in comparison to the leaves of the plants cultivated in the reference HPS-lamp system.

Based on additional fluorescence parameters of chlorophyll a, it may be also concluded that the type of the applied plant illumination system and illumination parameters have a significant influence on the photosynthesis process (the bright phase) in the plug plant phase. This fact is exemplified by the parameter Fᵥ/F₀, which describes the amount of the oxygen released during the photosynthesis process. The highest value of this parameter, and thus the greatest oxygen release, was observed for plants in the plug plant phase cultivated under the plant illumination system according to the invention, with the use of illumination parameters PULS1, while the smallest oxygen release was observed for plants illuminated in the reference HPS-lamp system. After 55 days of vegetation, similar relationships may be observed: oxygen releases for plants cultivated under the plant illumination system according to the invention were higher than the release observed in the reference HPS-lamp system.

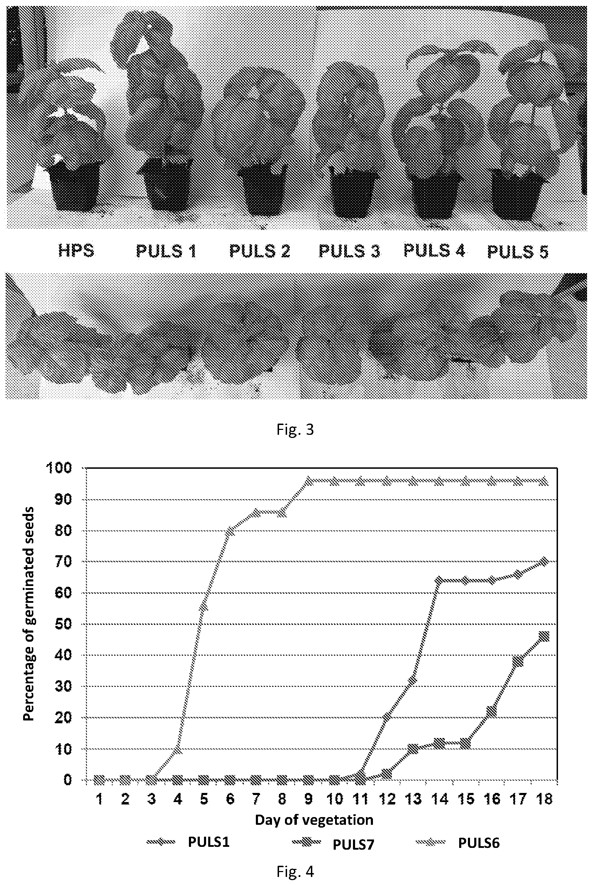

Fig. 3 shows a comparative photograph of Lettuce-leaved basil in its 55^{th} day of vegetation under different plant illumination systems, i.e. the plant illumination systems according to the invention (PULS1-5) and the reference (HPS) system. In the case of the plants in the 55^{th} day of vegetation, the shortest plants were obtained in the reference HPS-lamp system and in the plant illumination system according to the invention with the use of illumination parameters PULS2. The leaves were wavy to the greatest extent on the plants in the plant illumination systems PULS1 and PULS2, as well as in the reference HPS system (visual inspection, observation). The Lettuce-leaved basil under the plant illumination systems with the use of illumination parameters PULS1 and PULS5 was the highest, but the plants in the plant illumination system PULS1 at the same time showed small distances between leaf levels (internode lengths). The greatest distances between leave levels (internode lengths) were observed for the plants in the plant illumination system according to the invention with the use of illumination parameters PULS4 and PULS5. Different illuminations also caused differences in the number of leaf levels - HPS, PULS3, PULS5: 6 levels, PULS1: 8 levels, PULS2 and PULS4: 5 levels.

In sum, the growth/development of the basil was satisfactory for illumination conditions PULS1-5 in the plant illumination system according to the invention, and the plants reached similar or higher values of the tested parameters, which characterize among other things the growth or photosynthetic efficiency, in comparison to the values reached by the plants in the illumination conditions under the reference HPS-lamp system. In the cases of the more economic illumination parameters PULS2 and PULS4 in the plant illumination system of the invention, the values of the tested parameters were typically similar to the values reached by the plants in the reference HPS system. Higher values of the tested parameters (in comparison to the reference HPS system) were reached by the plants cultivated in illumination conditions PULS1 and PULS5 of the plant illumination system according to the invention.

### Example 2

The plant illumination system analogical to the first embodiment served to perform additional tests of the germination and growth of plants, with the use of the illumination parameters described in Table 4.

**Table 4- parameters of the applied illumination**

| | **P₁ [W]** | **P₂ [W]** | **t₁ [s]** | **t₂ [s]** | **T [cycles/min]** | **t₁/(t₁+t₂)** |
|---|---|---|---|---|---|---|
| **PULS1** | 200 | 20 | 4 | 8 | 5 | 0.333 |
| **PULS6** | 200 | 20 | 5 | 8 | 4.61 | 0.385 |
| **PULS7** | 200 | 20 | 3 | 5 | 7.5 | 0.375 |

where:
- P: ₁- Bright pulse power,
- P₂: - Dark pulse power,
- t₁: - Bright pulse duration time,
- t₂: - Dark pulse duration time,
- T: - Number of illumination cycles per minute.

The tests were performed for Lettuce-leaved basil and for Lena pepper, both dedicated for undercover cultivation. The tests were performed in closed, isolated vegetation chambers in controlled thermal conditions: temperature 25 °C ± 1 °C, photoperiod 12h/12h (day/night). Radiation intensity on the surface of the crop field was 270 µmol/m²/s. The seeds were sowed into a moist soil (the mixture: Ekoziem Universal Substrate for flowers and vegetables pH 6-7 (5L) + Substral Osmocote pH.

The following biometric tests were performed. When the test started, the germination time was identified by counting days from the sowing time and recording the number of the germinating sprouts. These data further served to calculate the germination percentage as a function of time.

In the 10^{th} day of vegetation (the plug plant phase), the plants were subjected to biometric measurements and growth observations. In the case of both basil and pepper, further biometric measurements, including measurements of plant height, the length of selected leaves and the number of leaves (leaf pairs), were performed in subsequent development phases (for the seedlings of the plug plants and for the plants in their 55^{th} day of vegetation). The plant height was measured from the base of the stem to the tip of the plant. In the case of the plants in the plug plant phase, the length parameters of the leaves were measured on leaf No. 1 (L1) of the pepper and on a leaf from the 1^{st} level (L1) of the basil. In the 55^{th} day of vegetation, the measurements were performed on leaf 5 (L5) for pepper and on a leaf from the 4^{th} level (pair) for basil, here referred to by symbol L4.

At the end of the test (55^{th} day of vegetation), the above-ground parts of basil and pepper were collected and weighed in order to measure the accumulation of fresh mass.

First, the germination dynamics were measured for Lena pepper in the illumination system according to the invention with the use of illumination parameters PULS1, PULS6 and PULS7. The results of this measurement are presented in the graph of Fig. 4. As can be observed, the fastest germination was observed for pepper seeds cultivated in the plant illumination system according to the invention with the use of illumination parameters PULS6, in which the first sprouts germinated already after 4 days. In the case of the other illumination parameters, i.e. PULS1 and PULS7, the first seed germinated after 11 and 12 days of vegetation, respectively. Differences were also observed regarding the number of germinated seeds. The greatest number of Lena pepper seeds were germinated already after 9 days of vegetation at illumination parameters PULS6 (96%), while after 18 days of vegetation 70% of seeds were germinated at illumination parameters PULS1, and the smallest number of seeds were germinated after 18 days at illumination conditions PULS7 (below 50%).

Table 5 includes the biometric measurements of the plants (Lena pepper) in various growth phases. The measurement data were normalized to the data shown for illumination parameters PULS1, which was the object of tests in the first embodiment.

**Table 5 - measurement data for Lena pepper**

| | **PULS1** | **PULS6** | **PULS7** |
|---|---|---|---|
| **mₛ** | 1 | 1.02 | 0.19 |
| **hₛ** | 1 | 3.5 | 0.44 |
| **lₛ (L1)** | 1 | 4.17 | 0.37 |
| **hᵣ** | 1 | 1.88 | 0.59 |
| **lᵣ (L1)** | 1 | 1.17 | 0.87 |
| **m_{w}** | 1 | 1.87 | 0.8 |
| **h_{w}** | 1 | 1.36 | 0.91 |
| **l_{w} (L1)** | 1 | 1.41 | 0.78 |

where:
mₛ - fresh mass of the Lena pepper seedling,
hₛ - height of the Lena pepper seedling,
lₛ- leaf length of the Lena pepper seedling(first leaf - L1),
hᵣ - height of the Lena pepper plug plant,
lᵣ - leaf length of the Lena pepper plug plant (first leaf - L1),
h_{w} - plant height for Lena pepper in its 55^{th} day of vegetation,
lₛ - leaf lengths of the Lena pepper plants (first leaf - L1) in their 55^{th} day of vegetation,
m_{w} - fresh mass (biomass accumulation) of Lena pepper in its 55^{th} day of vegetation.

Referring to the measurement data showed in Table 5, the highest results of the measured biometric parameters were obtained for the seedlings cultivated in the plant illumination system with the use of illumination parameters PULS6. Significantly lower values for the fresh mass of the seedling, height of the seedling and leaf length were obtained for the Lena pepper cultivated in the plant illumination system with the use of illumination parameters PULS7 and PULS1. The obtained seedlings also had different number of leaves. The greatest number of leaves was observed for the seedling sprouts cultivated with the use of illumination parameters PULS6: the number was more than 2-fold higher than in the case of the PULS1 and 4-fold higherthan in the case of illumination parameters PULS7. This fact implies faster plant development in the plant illumination system with the use of illumination parameters PULS6.

The highest pepper plug plants, on the other hand, were obtained in the plant illumination system with the use of illumination parameters PULS6. The plants cultivated with the use of illumination parameters PULS7 were significantly shorter than the plants in the other two cases. Significant differences were also observed for the length of the leaf developed on the pepper plug plant, which had the largest leaves in the case of illumination parameters PULS6. The smallest leaves were observed in plants cultivated in the plant illumination system with the use of illumination parameters PULS7.

At the same time, in the 55^{th} day of vegetation the highest values of the measured biometric parameters were observed for the pepper plants cultivated in the plant illumination system with the use of illumination parameters PULS6. These plants were significantly higher and had longer leaves than the plants cultivated in the plant illumination system with the use of illumination parameters PULS1 and PULS7, while the PULS7 pepper plants were the shortest. Similarly, the greatest accumulation of fresh biomass was observed also in the case of the plants cultivated in the plant illumination system with the use of illumination parameters PULS6.

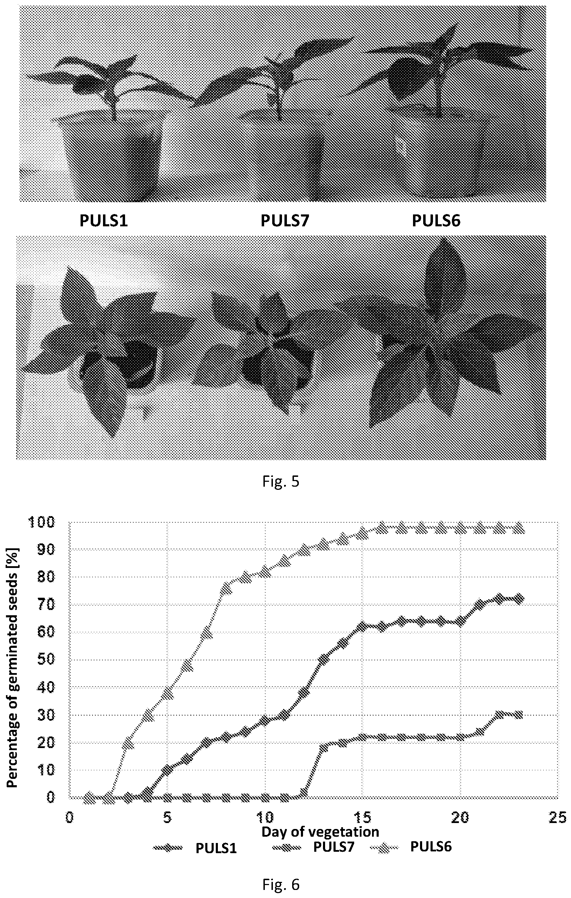

Fig. 5 shows a comparative photograph of Lena pepper in its 55^{th} day of vegetation under different plant illumination systems, i.e. the plant illumination systems according to the invention (PULS1, PULS6 and PULS7). Significant differences were observed for the plant habits and appearance of Lena pepper cultivated in the plant illumination system with the use of different illumination parameters. The Lena pepper plants cultivated with illumination parameters PULS6 were very well developed, with flower buds, with wide leaf habit, and with the thickest stem. The differences were also observed for the fifth leaf (L5). The plants cultivated with illumination parameters PULS6 had the largest fifth leaf and the leaf showed aging process, while the plants cultivated with illumination parameters PULS1 and PULS7 had young and small leaves, light-green in color. Differences regarding the number of leaves were also observed - PULS1: 8 leaves, PULS7: 8 leaves, and PULS6: 11 leaves and flower buds.

In sum, the plant illumination system with the use of illumination parameters PULS6 proved to be the most efficient and to most influence the development of Lena pepper during both the plug plant phase and the growth phase. These plants germinated at the fastest rate and without losses during germination (almost 100% germination). Moreover, the plants were higher and had more large leaves at all development stages. They were the only plants to develop flower buds before the end of the experiment.

Analogical tests were performed for Lettuce-leaved basil.

First, the germination dynamics were measured for Lettuce-leaved basil in the illumination system according to the invention with the use of illumination parameters PULS1, PULS6 and PULS7. The results of this measurement are presented in the graph of Fig. 6. As can be observed, the germination of basil seeds significantly differed for different illumination parameters in the plant illumination systems of the invention. With illumination parameters PULS6, 20% of the seeds were germinated already on the third day, while with illumination parameters PULS1, only 2% were germinated on the fourth day, and with illumination parameters PULS7 the first seeds were germinated after as long as 11 days. The greatest number of seeds germinated in the case of illumination parameters PULS6, reaching 98% already on the 5^{th} day, while in the case of PULS1, only 64% were germinated on the 18^{th} day of vegetation, and in the case of PULS6 it was almost 22%.

Table 6 includes the biometric measurements of the plants (Lettuce-leaved basil) in various growth phases. The measurement data were normalized to the data shown for illumination parameters PULS1, which was the object of tests in the first embodiment.

**Table 6 - measurement data for Lettuce-leaved basil**

| | **PULS1** | **PULS6** | **PULS7** |
|---|---|---|---|
| **mₛ** | 1 | 1.57 | 1.14 |
| **hₛ** | 1 | 1.62 | 0.74 |
| **lₛ (L1)** | 1 | 1.31 | 0.96 |
| **hᵣ** | 1 | 1.16 | 0.83 |
| **lᵣ (L1)** | 1 | 1.01 | 1.08 |
| **m_{w}** | 1 | 1.05 | 0.95 |
| **h_{w}** | 1 | 1.16 | 0.93 |
| **l_{w} (L1)** | 1 | 1.11 | 0.85 |

where:
mₛ- fresh mass of the Lettuce-leaved basil seedling,
hₛ - height of the Lettuce-leaved basil seedling,
lₛ- leaf length of the Lettuce-leaved basil seedling (first leaf - L1),
hᵣ - height of the Lettuce-leaved basil plug plant,
lᵣ - leaf length of the Lettuce-leaved basil plug plant (first leaf - L1),
h_{w} - plant height for Lettuce-leaved basil in its 55^{th} day of vegetation,
lₛ - leaf lengths of the Lettuce-leaved basil plants (first leaf - L1) in their 55^{th} day of vegetation,
m_{w} - fresh mass (biomass accumulation) for Lettuce-leaved basil in its 55^{th} day of vegetation.

Referring to the measurement data presented in Table 6, the basil seedlings cultivated in the plant illumination system according to the invention with the use of illumination parameters PULS6 had significantly greater fresh mass, seedling height and leaf length than the seedling sprouts in the other two cases. Also the seedlings cultivated with illumination parameters PULS6 had significantly more leaves than the seedlings cultivated with illumination parameters PULS1 and PULS7. The Lettuce-leaved seedlings cultivated in the plant illumination system with the use of illumination parameters PULS6 were more developed and reached the stage of a plug plant faster.

In the case of the Lettuce-leaved basil plug plants, significant differences were observed for all of the measured biometric parameters. The plug plants obtained in the plant illumination system with the use of illumination parameters PULS6 were significantly higher, and had longer leaves than the plug plants of Lettuce-leaved basil in the other two cases. The plug plant of Lettuce-leaved basil with illumination parameters PULS7 was the smallest, but had leaves of comparable length to the plug plant cultivated at illumination parameters PULS1.

At the plug plant stage, the largest Lettuce-leaved basil plants, i.e. the highest and with large leaves, were obtained in the plant illumination system with the use of illumination parameters PULS6. These plug plants were the highest, with lateral leaves in the first and second level, which fact was not observed on seedlings cultivated with illumination parameters PULS1 and PULS7. The Lettuce-leaved basil plug plants also differed in the number of leaf levels - PULS1: 3 levels, PULS7: 3 levels, PULS6: 4 levels.

As in the case of the Lettuce-leaved basil plug plants, significant differences were observed for all of the measured biometric parameters in the 55^{th} day of vegetation. The largest plants, in terms of height and leaf length, were Lettuce-leaved basil plants cultivated in the plant illumination system with the use of illumination parameters PULS6.

The Lettuce-leaved basil plants cultivated in the plant illumination system with the use of illumination parameters PULS6 also accumulated the most biomass.

Fig. 7 shows a comparative photograph of Lettuce-leaved basil in its 55^{th} day of vegetation under different plant illumination systems, i.e. the plant illumination systems according to the invention (PULS1, PULS6 and PULS7). Significant differences were observed for the plant habits and appearance of Lettuce-leaved basil cultivated in the plant illumination system with the use of different illumination parameters. The plants cultivated in the plant illumination system with the use of illumination parameters PULS6 were elongated, with larger gaps between the levels, but had large leaves and lateral shoots in each level. The plants cultivated with the use of illumination parameters PULS1 and PULS7 were smaller, but thicker, with smaller gaps between the levels, while the lateral shoots were observed only in the first level, for the basil cultivated under the plant illumination system with the use of illumination parameters PULS6. Moreover, the plants also differed in the number of levels - PULS1: 5 levels, PULS7: 5 levels, PULS6: 6 levels.

In sum, the germination stage and the young seedlings stage were found to be advantageously influenced by the plant illumination systems of the invention, especially by those with the use of illumination parameters PULS6. The germination is very fast and evenly distributed. The seedlings were significantly higher and had much larger leaves. In both phases (plug plant and growth), the best biometric parameters were observed for the plants in the plant illumination system with the use of illumination parameters PULS6. The Lettuce-leaved basil was higher, with large leaves and with primordia of lateral shoots. These plants also accumulated the most biomass.

The plant illumination system according to the invention provided advantages by accelerating the germination and reducing the losses in the germination, with the highest germination percentage. Moreover, during the plug plant and growth phases, the plants were higher and had a greater number of leaves. Additionally, the plants matured faster, allowing faster fructification (as in the case of the pepper).

### Example 3

The plant illumination system analogical to the first embodiment served to perform additional tests of the germination and growth of plants, for Red rubin basil and for great basil. The tests were performed for similar cultivation conditions, with the same photoperiod and soil as in the previous preferred embodiments.

The illumination parameters corresponded to the lighting frequency designated as PULS1, i.e. with the bright pulse duration time t₁ being 4 sec. and with the dark pulse duration time t₂ being 8 sec. The following light sources were used in this preferred embodiment: (1) bright pulse power 200 W (the photosynthetic photon flux (PPF) 680 µmol/s), dark pulse power 20 W (PPF 59 µmol/s), (2) bright pulse power 200 W (PPF 680 µmol/s), dark pulse power 40 W (PPF 131 µmol/s), and (3) bright pulse power 200 W (PPF 680 µmol/s), dark pulse power 60 W (PPF 195 µmol/s). The light sources were hung over the plants at a height providing 250 µmol/s/m². The tests demonstrated that each of the above illumination parameters provided evenly distributed germination, similar photosynthesis efficiency in the plug plant phase, and similar biometric parameters at each plant development stage.

## Claims

1. A plant illumination method in which light pulses are delivered to the plants in illumination cycles from a light source at any stage of plant development, one illumination cycle comprises a bright pulse having a duration time t₁ and a dark pulse having a duration time t₂, **characterized in that** an illumination cycle frequency is in the range of 2 cycles/minute to 6 cycles/minute, and the ratio between the bright pulse duration time t₁ and the duration time of the cycle t₁+t₂ is in the range of 0.25 to 0.5.

2. The plant illumination method as claimed in claim 1, **characterized in that** the bright pulse has an intensity corresponding to 90% - 100% of the maximum power of the light source, and the dark pulse has an intensity corresponding to 7% - 30% of the maximum power of the light source.

3. The plant illumination method as claimed in claim 1 or 2, **characterized in that** the light source maximum power emits a photosynthetic photon flux of 680 µmol/s and the photosynthetic photon flux density of the light source is in the range of 216 to 370 µmol/m²/s.

4. The plant illumination method according to any of claims 1 - 3, **characterized in that** the daily photoperiod is in the range of 12 hours to 24 hours.

5. The plant illumination method according to any of claims 1 - 4, **characterized in that** the bright pulse duration time t₁ is 4 seconds and the dark pulse duration time t₂ is 8 seconds, or the bright pulse duration time t₁ is 8 seconds and the dark pulse duration t₂ is 8 seconds, or the bright pulse duration time t₁ is 4 seconds and the dark pulse duration time t₂ is 12 seconds, or the bright pulse duration time t₁ is 5 seconds and the dark pulse duration time t₂ is 5 seconds, or the bright pulse duration time t₁ is 10 seconds and the dark pulse duration time t₂ is 20 seconds, or the bright pulse duration time t₁ is 5 seconds and the dark pulse duration time t₂ is 8 seconds.

6. The plant illumination method according to any of claims 1 - 5, **characterized in that** the light emitted from the light source has a wavelength in the range of 380 to 780 nm.

7. The plant illumination method according to any of claims 1 - 6, **characterized in that** the plant illumination pulses have a substantially rectangular waveform.

8. A plant illumination system comprising at least one light source connected to a power supply unit which includes a control means for providing cyclical operation of the light source, one illumination cycle comprises a bright pulse having a duration time t₁ and a dark pulse having a duration time t₂, **characterized in that** an illumination cycle frequency is in the range of 2 cycles/minute to 6 cycles/minute, and the ratio between the bright pulse duration time t₁ and the duration time of the cycle t₁+t₂ is in the range of 0.25 to 0.5.

9. The plant illumination system as claimed in claim 8, **characterized in that** the light source is a LED lamp.

10. The plant illumination system as claimed in claim 8 or 9, **characterized in that** the bright pulse has an intensity corresponding to 90% - 100% of the maximum power of the light source, and the dark pulse has an intensity corresponding to 7% - 30% of the maximum power of the light source.

11. The plant illumination system according to any of claims 8 - 10, **characterized in that** the light source maximum power emits a photosynthetic photon flux of 680 µmol/s and the photosynthetic photon flux density of the light source is in the range of 216 to 370 µmol/m²/s.

12. The plant illumination system according to any of claims 8 - 11, **characterized in that** the daily photoperiod is in the range of 12 hours to 24 hours.

13. The plant illumination system according to any of claims 8 - 12, **characterized in that** the bright pulse duration time t₁ is 4 seconds and the dark pulse duration time t₂ is 8 seconds, or the bright pulse duration time t₁ is 8 seconds and the dark pulse duration t₂ is 8 seconds, or the bright pulse duration time t₁ is 4 seconds and the dark pulse duration time t₂ is 12 seconds, or the bright pulse duration time t₁ is 5 seconds and the dark pulse duration time t₂ is 5 seconds, or the bright pulse duration time t₁ is 10 seconds and the dark pulse duration time t₂ is 20 seconds, or the bright pulse duration time t₁ is 5 seconds and the dark pulse duration time t₂ is 8 seconds.

14. The plant illumination system according to any of claims 8 - 13, **characterized in that** the light emitted from the light source has a wavelength in the range of 380 to 780 nm.

15. The plant illumination system according to any of claims 8 - 14, **characterized in that** the plant illumination pulses have a substantially rectangular waveform.

## Patentansprüche

1. Ein Verfahren zur Pflanzenbeleuchtung, bei dem Lichtimpulse in Beleuchtungszyklen von einer Lichtquelle in einem beliebigen Stadium der Pflanzenentwicklung den Pflanzen zugeführt werden, wobei ein Beleuchtungszyklus einen hellen Impuls mit einer Zeitdauer t₁ und einen dunklen Impuls mit einer Zeitdauer t₂ umfasst, **dadurch gekennzeichnet, dass** eine Frequenz des Beleuchtungszyklus im Bereich von 2 Zyklen/Minute bis 6 Zyklen/Minute liegt und das Verhältnis zwischen der Zeitdauer des hellen Impulses t₁ und der Zeitdauer des Zyklus t₁+t₂ im Bereich von 0,25 bis 0,5 liegt.

2. Das Verfahren zur Pflanzenbeleuchtung nach Anspruch 1, **dadurch gekennzeichnet, dass** der helle Impuls eine Intensität aufweist, die 90% - 100% der maximalen Leistung der Lichtquelle entspricht, und der dunkle Impuls eine Intensität aufweist, die 7% - 30% der maximalen Leistung der Lichtquelle entspricht.

3. Das Verfahren zur Pflanzenbeleuchtung nach Anspruch 1 oder 2, **dadurch gekennzeichnet, dass** die maximale Leistung der Lichtquelle einen photosynthetischen Photonenfluss von 680 µmol/s emittiert und die photosynthetische Photonenflussdichte der Lichtquelle im Bereich von 216 bis 370 µmol/m²/s liegt.

4. Das Verfahren zur Pflanzenbeleuchtung nach einem der Ansprüche 1 - 3, **dadurch gekennzeichnet, dass** die tägliche Photoperiode im Bereich von 12 Stunden bis 24 Stunden liegt.

5. Das Verfahren zur Pflanzenbeleuchtung nach einem der Ansprüche 1 - 4, **dadurch gekennzeichnet, dass** die Zeitdauer des hellen Impulses t₁ 4 Sekunden beträgt und die Zeitdauer des dunklen Impulses t₂ 8 Sekunden beträgt, oder die Zeitdauer des hellen Impulses t₁ 8 Sekunden beträgt und die Zeitdauer des dunklen Impulses t₂ 8 Sekunden beträgt, oder die Zeitdauer des hellen Impulses t₁ 4 Sekunden beträgt und die Zeitdauer des dunklen Impulses t₂ 12 Sekunden beträgt, oder die Zeitdauer des hellen Impulses t₁ 5 Sekunden beträgt und die Zeitdauer des dunklen Impulses t₂ 5 Sekunden beträgt, oder die Zeitdauer des hellen Impulses t₁ 10 Sekunden beträgt und die Zeitdauer des dunklen Impulses t₂ 20 Sekunden beträgt, oder die Zeitdauer des hellen Impulses t₁ 5 Sekunden beträgt und die Zeitdauer des dunklen Impulses t₂ 8 Sekunden beträgt.

6. Das Verfahren zur Pflanzenbeleuchtung nach einem der Ansprüche 1 - 5, **dadurch gekennzeichnet, dass** das von der Lichtquelle emittierte Licht eine Wellenlänge im Bereich von 380 bis 780 nm aufweist.

7. Das Verfahren zur Pflanzenbeleuchtung nach einem der Ansprüche 1 - 6, **dadurch gekennzeichnet, dass** die Pflanzenbeleuchtungsimpulse eine im Wesentlichen rechteckige Wellenform aufweisen.

8. Ein System zur Pflanzenbeleuchtung, umfassend mindestens eine Lichtquelle, die mit einer Stromversorgungseinheit verbunden ist, die Steuermittel zum Bereitstellen eines zyklischen Betriebs der Lichtquelle umfasst, wobei ein Beleuchtungszyklus einen hellen Impuls mit einer Zeitdauer t₁ und einen dunklen Impuls mit einer Zeitdauer t₂ umfasst, **dadurch gekennzeichnet, dass** eine Frequenz des Beleuchtungszyklus im Bereich von 2 Zyklen/Minute bis 6 Zyklen/Minute liegt und das Verhältnis zwischen der Zeitdauer des hellen Impulses t₁ und der Zeitdauer des Zyklus t₁+t₂ im Bereich von 0,25 bis 0,5 liegt.

9. Das System zur Pflanzenbeleuchtung nach Anspruch 8, **dadurch gekennzeichnet, dass** die Lichtquelle eine LED-Lampe ist.

10. Das System zur Pflanzenbeleuchtung nach Anspruch 8 oder 9, **dadurch gekennzeichnet, dass** der helle Impuls eine Intensität aufweist, die 90% - 100% der maximalen Leistung der Lichtquelle entspricht, und der dunkle Impuls eine Intensität aufweist, die 7% - 30% der maximalen Leistung der Lichtquelle entspricht.

11. Das System zur Pflanzenbeleuchtung nach einem der Ansprüche 8 - 10, **dadurch gekennzeichnet, dass** die maximale Leistung der Lichtquelle einen photosynthetischen Photonenfluss von 680 µmol/s emittiert und die photosynthetische Photonenflussdichte der Lichtquelle im Bereich von 216 bis 370 µmol/m²/s liegt.

12. Das System zur Pflanzenbeleuchtung nach einem der Ansprüche 8 - 11, **dadurch gekennzeichnet, dass** die tägliche Photoperiode im Bereich von 12 Stunden bis 24 Stunden liegt.

13. Das System zur Pflanzenbeleuchtung nach einem der Ansprüche 8 - 12, **dadurch gekennzeichnet, dass** die Zeitdauer des hellen Impulses t₁ 4 Sekunden beträgt und die Zeitdauer des dunklen Impulses t₂ 8 Sekunden beträgt, oder die Zeitdauer des hellen Impulses t₁ 8 Sekunden beträgt und die Zeitdauer des dunklen Impulses t₂ 8 Sekunden beträgt, oder die Zeitdauer des hellen Impulses t₁ 4 Sekunden beträgt und die Zeitdauer des dunklen Impulses t₂ 12 Sekunden beträgt, oder die Zeitdauer des hellen Impulses t₁ 5 Sekunden beträgt und die Zeitdauer des dunklen Impulses t₂ 5 Sekunden beträgt, oder die Zeitdauer des hellen Impulses t₁ 10 Sekunden beträgt und die Zeitdauer des dunklen Impulses t₂ 20 Sekunden beträgt, oder die Zeitdauer des hellen Impulses t₁ 5 Sekunden beträgt und die Zeitdauer des dunklen Impulses t₂ 8 Sekunden beträgt.

14. Das System zur Pflanzenbeleuchtung nach einem der Ansprüche 8 - 13, **dadurch gekennzeichnet, dass** das von der Lichtquelle emittierte Licht eine Wellenlänge im Bereich von 380 bis 780 nm aufweist.

15. Das System zur Pflanzenbeleuchtung nach einem der Ansprüche 8 - 14, **dadurch gekennzeichnet, dass** die Pflanzenbeleuchtungsimpulse eine im Wesentlichen rechteckige Wellenform aufweisen.

## Revendications

1. Procédé d'éclairage de plante dans lequel des impulsions lumineuses sont délivrées aux plantes dans des cycles d'éclairage à partir d'une source de lumière à un stade quelconque du développement de la plante, un cycle d'éclairage comprenant une impulsion lumineuse ayant un temps de durée t₁ et une impulsion sombre ayant un temps de durée t₂, **caractérisé en ce qu'**une fréquence de cycle d'éclairage est dans la plage de 2 cycles/minute à 6 cycles/minute, et le rapport entre le temps de durée d'impulsion lumineuse t₁ et le temps de durée du cycle t₁+t₂ est dans la plage de 0,25 à 0,5.

2. Procédé d'éclairage de plante selon la revendication 1, **caractérisé en ce que** l'impulsion lumineuse a une intensité correspondant à 90 % à 100 % de la puissance maximale de la source lumineuse, et l'impulsion sombre a une intensité correspondant à 7 % à 30 % de la puissance maximale de la source lumineuse.

3. Procédé d'éclairage de plante selon la revendication 1 ou 2, **caractérisé en ce que** la puissance maximale de la source de lumière émet un flux de photons photosynthétiques de 680 µmol/s et la densité de flux de photons photosynthétiques de la source de lumière est dans la plage de 216 à 370 µmol/m²/s.

4. Procédé d'éclairage de plante selon l'une quelconque des revendications 1 à 3, **caractérisé en ce que** la photopériode quotidienne est dans la plage de 12 heures à 24 heures.

5. Procédé d'éclairage de plante selon l'une quelconque des revendications 1 à 4, **caractérisé en ce que** le temps de durée d'impulsion lumineuse t₁ est de 4 secondes et le temps de durée d'impulsion sombre t₂ est de 8 secondes, ou le temps de durée d'impulsion lumineuse t₁ est de 8 secondes et la durée d'impulsion sombre t₂ est de 8 secondes, ou le temps de durée d'impulsion lumineuse t₁ est de 4 secondes et le temps de durée d'impulsion sombre t₂ est de 12 secondes, ou le temps de durée d'impulsion lumineuse t₁ est de 5 secondes et le temps de durée d'impulsion sombre t₂ est de 5 secondes, ou le temps de durée d'impulsion lumineuse t₁ est de 10 secondes et le temps de durée d'impulsion sombre t₂ est de 20 secondes, ou le temps de durée d'impulsion lumineuse t₁ est de 5 secondes et le temps de durée d'impulsion sombre t₂ est de 8 secondes.

6. Procédé d'éclairage de plante selon l'une quelconque des revendications 1 à 5, **caractérisé en ce que** la lumière émise à partir de la source de lumière a une longueur d'onde dans la plage de 380 à 780 nm.

7. Procédé d'éclairage de plante selon l'une quelconque des revendications 1 à 6, **caractérisé en ce que** les impulsions d'éclairage de plante ont une forme d'onde sensiblement rectangulaire.

8. Système d'éclairage de plante comprenant au moins une source de lumière connectée à une unité d'alimentation en énergie qui comprend un moyen de commande pour fournir un fonctionnement cyclique de la source de lumière, un cycle d'éclairage comprenant une impulsion lumineuse ayant un temps de durée t₁ et une impulsion sombre ayant un temps de durée t₂, **caractérisé en ce qu'une** fréquence de cycle d'éclairage est dans la plage de 2 cycles/minute à 6 cycles/minute, et le rapport entre le temps de durée d'impulsion lumineuse t₁ et le temps de durée du cycle t₁+t₂ est dans la plage de 0,25 à 0,5.

9. Système d'éclairage de plante selon la revendication 8, **caractérisé en ce que** la source de lumière est une lampe LED.

10. Système d'éclairage de plante selon la revendication 8 ou 9, **caractérisé en ce que** l'impulsion lumineuse a une intensité correspondant à 90 % à 100 % de la puissance maximale de la source lumineuse, et l'impulsion sombre a une intensité correspondant à 7 % à 30 % de la puissance maximale de la source lumineuse.

11. Système d'éclairage de plante selon l'une quelconque des revendications 8 à 10, **caractérisé en ce que** la puissance maximale de la source de lumière émet un flux de photons photosynthétiques de 680 µmol/s et la densité de flux de photons photosynthétiques de la source de lumière est dans la plage de 216 à 370 µmol/m²/s.

12. Système d'éclairage de plante selon l'une quelconque des revendications 8 à 11, **caractérisé en ce que** la photopériode quotidienne est dans la plage de 12 heures à 24 heures.

13. Système d'éclairage de plante selon l'une quelconque des revendications 8-12, **caractérisé en ce que** le temps de durée d'impulsion lumineuse t₁ est de 4 secondes et le temps de durée d'impulsion sombre t₂ est de 8 secondes, ou le temps de durée d'impulsion lumineuse t₁ est de 8 secondes et la durée d'impulsion sombre t₂ est de 8 secondes, ou le temps de durée d'impulsion lumineuse t₁ est de 4 secondes et le temps de durée d'impulsion sombre t₂ est de 12 secondes, ou le temps de durée d'impulsion lumineuse t₁ est de 5 secondes et le temps de durée d'impulsion sombre t₂ est de 5 secondes, ou le temps de durée d'impulsion lumineuse t₁ est de 10 secondes et le temps de durée d'impulsion sombre t₂ est de 20 secondes, ou le temps de durée d'impulsion lumineuse t₁ est de 5 secondes et le temps de durée d'impulsion sombre t₂ est de 8 secondes.

14. Système d'éclairage de plante selon l'une quelconque des revendications 8 à 13, **caractérisé en ce que** la lumière émise à partir de la source de lumière a une longueur d'onde dans la plage de 380 à 780 nm.

15. Système d'éclairage de plante selon l'une quelconque des revendications 8 à 14, **caractérisé en ce que** les impulsions d'éclairage de plante ont une forme d'onde sensiblement rectangulaire.
